# EUROPÄISCHE PATENTSCHRIFT

(11) **EP 2 677 944 B1**
(45) Veröffentlichungstag und Bekanntmachung des Hinweises auf die Patenterteilung: **19.12.2018**
(21) Anmeldenummer: 12710653.2
(22) Anmeldetag: 22.02.2012
(51) Int. Cl.: A61B 17/12

(54) **IMPLANTAT INSBESONDERE FÜR DIE OKKLUDIERUNG VON BIFURKATIONSANEURYSMEN**
IMPLANT, ESPECIALLY FOR THE OCCLUSION OF BIFURCATION ANEURYSMS
IMPLANT UTILISÉ NOTAMMENT POUR L'OCCLUSION D'ANÉVRISMES DE BIFURCATION

(30) Priorität: 22.02.2011 DE 102011011869
(43) Veröffentlichungstag der Anmeldung: 01.01.2014
(73) Patentinhaber: Phenox GmbH, 44801 Bochum (DE)
(72) Erfinder: HANNES, Ralf, 44137 Dortmund (DE); MONSTADT, Hermann, 44797 Bochum (DE)
(74) Vertreter: Thiel, Christian
(86) Internationale Anmeldenummer: PCT/EP2012/000772
(87) Internationale Veröffentlichungsnummer: WO 2012/113554

(56) Entgegenhaltungen:
- WO-A1-01/93782
- WO-A1-02/00139
- WO-A1-2005/117718
- WO-A1-2010/028314
- WO-A2-02/054980
- WO-A2-2006/052322
- WO-A2-2006/052322
- DE-A1-102008 028 308
- DE-A1-102008 028 308
- US-A1- 2003 055 440
- US-A1- 2005 096 728
- US-A1- 2007 198 075
- US-A1- 2007 198 075
- US-A1- 2007 203 567
- US-A1- 2007 270 902
- US-A1- 2008 125 855
- US-A1- 2009 264 914
- US-B2- 6 818 013

## Beschreibung

Die Erfindung betrifft ein Implantat zum Einsatz bei der Okkludierung von Aneurysmen in Gefäßverzweigungen, insbesondere von Bifurkationsaneurysmen. Ein solches Implantat wird mit Hilfe eines Katheters und Führungsdrahts an den Implantatsort gebracht und dort dauerhaft implantiert. Entsprechend betrifft die Erfindung auch ein solches Implantat, implantationsfertig angekoppelt an einen Führungsdraht.

Arteriovenöse Fehlbildungen können in einem Patienten zu erheblichen Beeinträchtigungen und Gefährdungen bis hin zum Tode führen. Dies gilt insbesondere auch für Aneurysmen, insbesondere dann, wenn sie im zerebralen Bereich auftreten. In der Regel versucht man derartige Fehlbildungen durch Implantate zu verschließen. Solche Implantate werden in der Regel auf endovaskulärem Weg mit Hilfe von Kathetern gesetzt.

Insbesondere bei zerebralen Aneurysmen hat sich die Implantierung von Platinspiralen bewährt, die das Aneurysma mehr oder weniger vollständig ausfüllen, den Bluteinstrom weitgehend blockieren und dazu führen, dass sich ein lokaler Thrombus ausbildet, der das Aneurysma ausfüllt und letztlich verschließt. Diese Behandlungsmethode ist allerdings nur bei Aneurysmen geeignet, die über einen relativ engen Zugang zum Gefäßsystem verfügen, sogenannten Beerenaneurysmen. Bei Aussackungen von Blutgefäßen, die über einen weiten Zugang zum Gefäß verfügen, drohen die implantierten Spiralen wieder ausgeschwemmt zu werden. Diese werden dann in anderen Bereich des Gefäßsystems gelagert und können dort weitere Schäden herbeiführen.

In solchen Fällen wurde bereits vorgeschlagen, eine Art Stent zu setzen, der die Öffnung des Aneurysmas "vergittert" und dadurch die Ausschwemmung der Okklusionsspiralen verhindert. Derartige Stents, die über eine relativ weitmaschige Wandung verfügen, werden bereits bei einigen Formen von Aneurysmen eingesetzt.

Die US 2007/198075 A1 beschreibt ein Implantat mit den Merkmalen des Oberbegriffs des Anspruchs 1. Aus DE 10 2008 028 308 A1 geht die Verwendung von Drahtschlaufen als Widerlager und Zentriermittel zur Zentrierung des Implantats im Aneurysmahals hervor.

Gefäßverzweigungen, insbesondere Gefäßbifurkationen, sind ein relativ häufiges Phänomen. Das durch eine Arterie im Bereich einer Gabelung auf die Stirnwand aufprallende Blut führt - im Fall einer Schwächung der Gefäßwandung - schnell zu einer Aussackung, die sich dann schnell erweitert. Solche Bifurkationsaneurysmen haben häufig einen weiten Hals, der das Einbringen von Okklusionsspiralen unmöglich macht.

Gleichzeitig fehlt es an Stentstrukturen, die geeignet sind, im Bereich einer Gefäßverzweigung eine "Vergitterung" des Aneurysmaeingangs herbeizuführen. Solche Stents sind nur mit Schwierigkeiten und unter hohen Kosten herstellbar und auch außerordentlich schwer zu platzieren.

Unter diesem Gesichtspunkt ist es Aufgabe der Erfindung, ein Implantat bereitzustellen, das geeignet ist, im Bereich insbesondere von Bifurkationsaneurysmen eingesetzt zu werden und dort den Eingang eines Aneurysmas zu "vergittern". Mit anschließend eingebrachten Okklusionsspiralen kann dann das Aneurysma stillgelegt werden.

Eine solche "Vergitterung" ist auch im Sinne einer Beeinflussung des Blutstroms denkbar, um die Anzahl der Okklusionsspiralen zu reduzieren oder auf Null zu setzen.

Diese Aufgabe wird mit einem Implantat mit einer Maschenstruktur gelöst, welches - von proximal nach distal - die Abschnitte (a) bis (d) aufweist:
(a) einen sich verjüngenden proximalen Abschnitt, in dem die Maschenstruktur zu einem oder mehreren Kupplungselementen zusammengeführt ist,
(b) einen Fixierabschnitt, mit dem das Implantat an einer Gefäßwand abstützbar ist,
(c) einen durchlässigen Abschnitt mit einer größeren Maschenweite als im Abschnitt (b) für den Bereich der Gefäßbifurkation und
(d) einen distalen Abschnitt, in dem das Implantat gegenüber den Abschnitt (b) erweitert ist und der zur Platzierung im Aneurysma bestimmt ist,
wobei zwischen den Abschnitten (c) und (d) eine Trennzone angeordnet ist, wobei die Trennzone Trennelemente aus quer zum Implantat im Wesentlichen in einer Ebene verlaufenden Filamentenaufweist und der distale Abschnitt aus einer Mehrzahl von mit dem Abschnitt verbundenen Schlaufen besteht, die gerundete und atraumatische Nasen am distalen Ende aufweisen, wobei der distale Abschnitt trompetenförmig oder korbförmig erweitert ist.

Die Begriffe "proximal" und "distal" sind so zu verstehen, dass sie zum Führungsdraht und somit zum Katheter und behandelnden Arzt weisende Teile des Implantats bezeichnen (proximal) bzw. vom Führungsdraht oder behandelnden Arzt wegweisende Teile (distal). Proximal ist damit führungsdrahtseitig und distal führungsdrahtabgewandt.

Bei dem erfindungsgemäßen Implantat handelt es sich um ein Implantat mit einer Maschenstruktur, die aus einem Geflecht einzelner Drähte bestehen kann, eine aus einem Rohr geschnittene Maschenstruktur aufweisen kann oder eine Kombination von beidem ist. Insoweit handelt es sich bei dem Implantat weitgehend um einen Stent oder ein stentähnliches Gebilde, das sich durch die besondere Art seines Einsatzes und Aufbaus auszeichnet.

Das erfindungsgemäße Implantat ist in insgesamt vier Abschnitte gegliedert, die Abschnitte (a) bis (d), von proximal nach distal gesehen.

Der Abschnitt (a) ist ein sich verjüngender proximaler Abschnitt, in dem die Maschenstruktur zu einem oder mehreren Kupplungselementen zusammengeführt ist. Die Kupplungselemente befinden sich vorzugsweise an der Peripherie, d. h. kommen im implantierten Zustand an der Gefäßwandung zum Liegen. Eine zentrierte Anordnung ist auch aus applikationstechnischen Gründen nicht sinnvoll; die periphere Anordnung des oder der Kupplungselemente erleichtert bei Fehlplatzierungen das Rückziehen des Implantats in den Platzierungskatheter. Bevorzugt sind Ausführungsformen mit einem oder zwei Kupplungselementen.

Abschnitt (b) ist ein Fixierabschnitt, mit dem sich das Implantat an der Gefäßwand des Blut heranführenden Gefäßes abstützt. In diesem Bereich ist das Gefäß nicht geschädigt und geeignet, mit einer Stentwandung beaufschlagt zu werden. Bei selbstexpandierenden Implantaten legt sich der Abschnitt (b) nach der Freisetzung aus dem Katheter selbständig an die Gefäßwandung an, bei ballonplatzierbaren Implantat wird das Implantat in diesem Bereich über ein Platzierungsballon aufgeweitet und gegen die Gefäßwand gepresst.

Abschnitt (c) ist ein durchlässiger Abschnitt, der eine größere Maschenweite als Abschnitt (b) aufweist und der in den Bereich der eigentlichen Gefäßbifurkation gesetzt wird. Eine größere Maschenweite erlaubt einen mehr oder weniger ungehemmten Blutfluss durch die Maschen hindurch in die abführenden Gefäßzweige.

Der distale Abschnitt (d) ist gegenüber dem Abschnitt (b) und zumeist auch gegenüber dem Abschnitt (c) nach außen erweitert. Er dient der Platzierung im Aneurysma selbst, an dessen sich erweiternde Wandung er sich anpassen soll.

Zwischen den Abschnitten (c) und (d) ist eine Trennzone angeordnet, die insbesondere der Rückhaltung von in das Bifurkationsaneurysma angebrachte Okklusionsmittel dienen soll.

Die Erweiterung des Abschnitts (d) des erfindungsgemäßen Implantats ist trompetenförmig oder korbförmig ausgebildet. Eine solche Erweiterung wird durch die Ausbildung von Schlaufen erzeugt . Eine solche schlaufenförmige Erweiterung weist in der Regel wenigstens zwei Schlaufen auf, insbesondere drei Schlaufen oder mehr.

Bei den Schlaufen kann es sich um entsprechend geformte Drahtelemente handeln, sie können aber auch, soweit das Implantat aus einem Rohr geschnitten ist, entsprechend durch Laserschneiden des gleichen Rohrs erzeugt sein.

Die erfindungsgemäßen Implantate können aus herkömmlichen Stentmaterialien gefertigt sein, beispielsweise aus medizinischem Stahl oder Kobaltchromlegierungen, bestehen aber insbesondere aus Formgedächtnismaterial, etwa Nitinol oder ternären Nickeltitanlegierungen.

Wie schon angesprochen, ist ein erfindungsgemäßes Implantat vorzugsweise zumindest partiell aus einem Rohr geschnitten, insbesondere aus einem Rohr aus einer Formgedächtnislegierung.

Die im erfindungsgemäßen Implantat vorgesehene Trennzone verläuft zwischen den Abschnitten (c) und (d). In diesem Zusammenhang ist festzustellen, dass der Abschnitt (c) zumindest in seinem distalen Ende bereits gegenüber dem Abschnitt (b) erweitert sein kann, was dann hilfreich ist, wenn das Bifurkationsaneurysma bereits Teile der "Prallwand" der Gefäßabzweigungen eingenommen hat. In diesem Fall muss der Eingangsteil des Aneurysmas für den abzweigenden Blutstrom freigehalten werden, so dass die Trennzone im Aneurysma selbst verläuft. Der bereits erweiterte Bereich des Abschnitts (c) geht dann, ggf. unter weiterer Aufweitung, in den Abschnitt (d) über. Auch hier liegt die Trennzone zwischen den Abschnitten (c) und (d). Bei einer sehr flachen Ausgestaltung des Abschnitts (d) kann die Trennzone auch mit dem Abschnitt (d) zusammenfallen.

Die Trennzone wird durch das Einziehen von Fasern, Fäden, dünnen Drähten, oder dergleichen Trennelemente ausgebildet, kann aber auch integraler Teil des Implantats in dem Sinne sein, dass es sich um aus dem Ausgangsrohr herausgeschnittene und entsprechend umgeformte Trennelemente, etwa Schlaufen oder Stege, handelt. Wesentlich ist, das diese Trennzone ihre Funktion erfüllt, nämlich in dem distalen Bereich (d) des Implantats eingeführte Okklusionsmittel, etwa Okklusionsspiralen, zuverlässig zurückzuhalten oder den Blutstrom so abzulenken, dass weitere Okklusionsmittel unnötig sind.

Wird die Trennzone durch das Einziehen von Fasern, Fäden oder dünnen Drähten ausgebildet, ist die Anordnung von Ösen im Bereich der Trennzone zweckmäßig. Beispielsweise können die Maschen des Abschnitts (d) mit entsprechenden Ösen ausgestattet sein, in die Nylonfäden kreuz- oder sternförmig eingeknotet werden.

Die Trennzone kann aber auch durch aus dem Rohrmaterial geschnittene Bögen gebildet werden, wobei die Maschen des Abschnitts (d) nach außen umgeformt werden und die Bögen der Trennzone in den Implantatkörper hinein umgebogen sind. Mindestens ein Bogen ist erforderlich. Bei zwei bis vier Bögen bilden diese ein stabiles Trennelement, das in ein Aneurysma eingebrachte Okklusionsmittel zuverlässig zurückhält.

Der distale Abschnitt (d) des erfindungsgemäßen Implantats ist insbesondere atraumatisch, weich und elastisch ausgebildet. Die Wandungen von Aneurysmen sind empfindlich und können bei Belastung reißen, was unter allen Umständen verhindert werden muss. Entsprechend muss insbesondere der distale Abschnitt (d) des erfindungsgemäßen Implantats atraumatisch gestaltet sein. Dies wird beispielsweise mit der Anordnung von Schlaufen erreicht, die sich dort, wo sie Kontakt mit der Aneurysmawandung bekommen, sanft an diese anschmiegen. Solche Schlaufen werden , wie auch die anderen Bereiche des Implantats, durch Laserschneiden aus einem Rohr generiert, können aber auch mittels angehefteter Drähte erzeugt werden, die beispielsweise mit dem Abschnitt (c) laserverschweißt sind. Diese Übergangszone deckt sich insbesondere mit der Trennzone, kann aber auch einen erweiterten Bereich des Abschnitts (c) darstellen, mit distal davon angeordneter Trennzone.

In jedem Fall ist es wichtig, im distalen Abschnitt (d) alle Drahtenden atraumatisch zusammenzuführen, um eine Perforation der Aneurysmawand zu verhindern.

Die Maschen im distalen Abschnitt (d) weisen am distalen Ende Nasen auf, welche naturgemäß aber ebenfalls gerundet sind und atraumatisch. Diese Nasen haben den Effekt, dass sich das Implantat in der gestreckten Form im Katheter einfacher d. h. mit geringerem Kraftaufwand, verschieben lässt.

Die erfindungsgemäßen Implantate können durchgängig die Form eines aus der Maschenstruktur gebildeten seitlich geschlossenen Rohrs haben, jedoch auch seitlich partiell oder durchgehend geschlitzt sein. Die Schlitzung kann achsparallel verlaufen oder schräg-/helixförmig. In solch einem Fall ist in den geschlitzten Bereichen die Maschenstruktur entsprechend der Gefäßform gerollt, etwa in der Form eines gerollten Segments eines Maschendrahtzauns. Bei der Implantation eines solchen geschlitzten Implantats erlaubt dies eine gute Anpassung an das Gefäßlumen, insbesondere des zuführenden Gefäßes, wobei eine geringfügige Unter- oder Überdeckung der seitlichen Ränder der Maschenstruktur in der Regel unproblematisch ist.

Bevorzugt ist eine partielle Schlitzung, die am distalen Abschnitt (d) endet. Eine solche Schlitzung erlaubt eine bessere Anpassung an den Gefäßverlauf, insbesondere im Bereich der Abschnitte (a) bis (c) und damit zu einer besseren Fixierung des Implantats im Gefäß. Überraschend hat sich gezeigt, dass die Schlitzung keinen negativen Einfluss auf die Radialkraft ausüben muss.

Die erfindungsgemäßen Implantate weisen in der Regel Markerelemente auf, die die Platzierung am Implantatsort erleichtern. Solche Markerelemente sind beispielsweise im Bereich des distalen Endes des Abschnitts (d) angeordnet, wobei sie bei zusammengeführten Drähten die Verbindungspunkte atraumatisch umformen können. Solche Markerelemente können aber auch in Form von Wicklungen an Drahtschlaufen vorliegen, oder als Manschetten im Übergangsbereich der Abschnitte (c) und (d). Für die Markerelemente kommen als Materialien insbesondere Platin und Platinlegierungen in Frage, beispielsweise eine Legierung aus Platin und Iridium, wie sie vielfach im Stand der Technik für Markerzwecke und als Material für Okklusionscoils eingesetzt wird.

Die Erfindung betrifft schließlich ein Implantat nach der vorstehenden Beschreibung, das an einen üblichen Führungsdraht angekoppelt ist. Diese Ankopplung kann beispielsweise durch Verbindungselemente erfolgen, die sich unter Einwirkung von elektrischem Strom elektrolytisch auflösen. Solche Verbindungselemente und Materialien sind insbesondere für die Ablösung von Okklusionsspiralen und Stents vielfach beschrieben. Auch eine mechanische Ablösung durch Kupplungselemente ist ohne Weiteres möglich, wobei die Kupplungselemente mit entsprechend angepassten Kupplungsteilen des Führungsdrahts zusammenwirken. Unter dem äußeren Zwang eines Katheters oder einer Hülle bleibt diese Verbindung intakt; nach Herausschieben des Implantats und der Kupplungsstelle aus dem Katheter oder der Umhüllung löst sich aber die Verbindung und setzt das Implantat mit den zum Implantat gehörigen Kupplungselementen frei.

Die erfindungsgemäßen Implantate werden mit Hilfe eines üblichen Katheters oder Mikrokatheters platziert; diese Technik ist allgemein erprobt und wird vielfach angewandt.

Die Erfindung wird durch die beiliegenden Abbildungen näher erläutert. Es zeigen:
- Figur 1: als Prinzipskizze ein Bifurkationsaneurysma;
- Figur 2: schematisch ein erfindungsgemäßes Implantat, implantiert in den Bereich einer Gefäßbifurkation mit Bifurkationsaneurysma;
- Figur 3: das erfindungsgemäße Implantat mit seinen Sektionen im Prinzip;
- Figur 4: ein erfindungsgemäßes Implantat, wie es gemäß Figur 2 eingesetzt werden kann;
- Figur 5: Varianten für den Abschnitt (d) eines erfindungsgemäßen Implantats;
- Figur 6: eine bevorzugte Ausführungsform eines erfindungsgemäßen Implantats, flächig ausgebreitet;
- Figur 7: eine weitere Ausführungsform eines erfindungsgemäßen Implantats gemäß Figur 6;
- Figur 8: Varianten eines erfindungsgemäßen Implantats mit schlaufenförmigen distalen Abschnitten (d);
- Figur 9: ein Bifurkationsaneurysma mit aus dem Aneurysmabereich abzweigenden Seitengefäßen und einen implantierten erfindungsgemäßen Implantat;
- Figur 10: in flächig ausgebreiteter Form Varianten erfindungsgemäßer Implantate;
- Figur 11: eine weitere Variante mit einwärts- und auswärtsweisenden Bögen im Abschnitt (d);
- Figur 12: eine weitere Variante mit Gelenkverbindern im Abschnitt (c); und
- Figur 13: eine weitere Variante eines erfindungsgemäßen Implantats mit erhöhter Flexibilität.

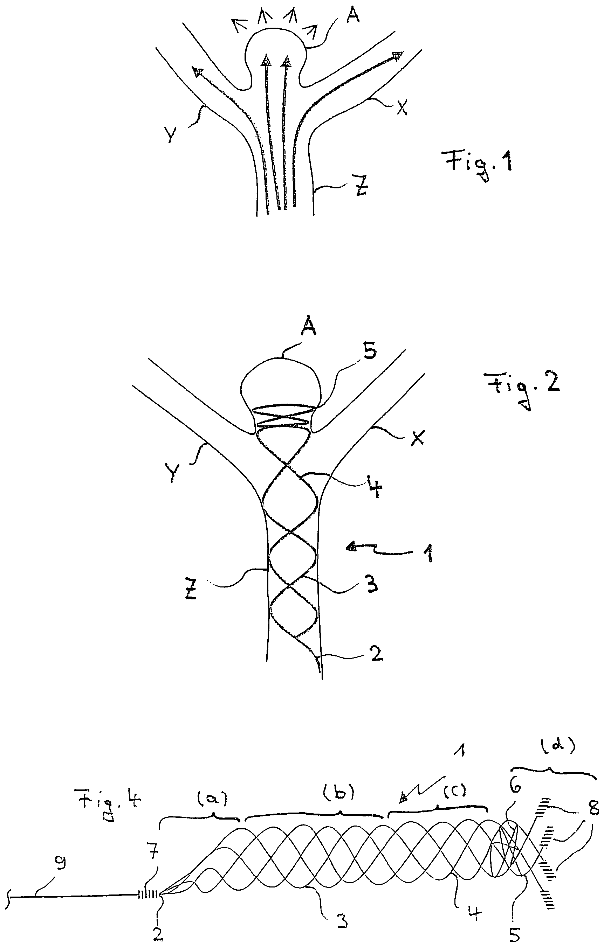

Figur 1 zeigt ein Bifurkationsaneurysma mit einem zuführenden Gefäß Z, zwei abführenden Gefäßen X und Y sowie dem in der Gabelung angeordneten Aneurysma A. Die langen Pfeile stellen den Blutstrom dar, der auf der Prallseite in das Aneurysma A einfließt und dort einen Druck nach außen ausübt, unter dem das Aneurysma sich erweitert (kleine Pfeile).

Figur 2 zeigt eine Gefäßkonstellation mit einem Aneurysma A, wie in Figur 1 beschrieben, mit einem darin angeordneten erfindungsgemäßen Implantat 1. Das Implantat hat ein proximales Ende 2, das das Kupplungselement aufweist und vor der Ablösung in den Führungsdraht (nicht dargestellt) übergeht. Das Implantat 1 ist über seine Maschen 3 an der Gefäßwand des zuführenden Gefäßes Z verankert und weist im Bereich der Bifurkation Maschen 4 mit einer größeren Maschenweite auf. Im Hals des Aneurysma ist ein distaler Bereich 5 dargestellt. Zwischen dem distalen Bereich 5 und dem Bereich mit den erweiterten Maschen 4 befindet sich eine Trennzone zur Rückhaltung von in das Aneurysma A nach Setzen des Implantats eingeführten Okklusionsmitteln.

Die erweiterten Maschen 4 im Bereich der Bifurkation erlauben es dem über das zuführende Gefäß Z einfließenden Blutstrom, in die Abzweigungen X und Y abzufließen, ohne groß behindert zu werden. Nach Setzen von hier nicht dargestellten Okklusionsmitteln im Aneurysma A ist der Blutstrom in das Aneurysma A so behindert, dass es dort zur Ausbildung eines Pfropfens und damit zur Stilllegung des Aneurysmas kommt. Alternativ kommt es zur Okklusion ohne Okklusionsmittel, sofern die Trennzone hinreichend dicht ist.

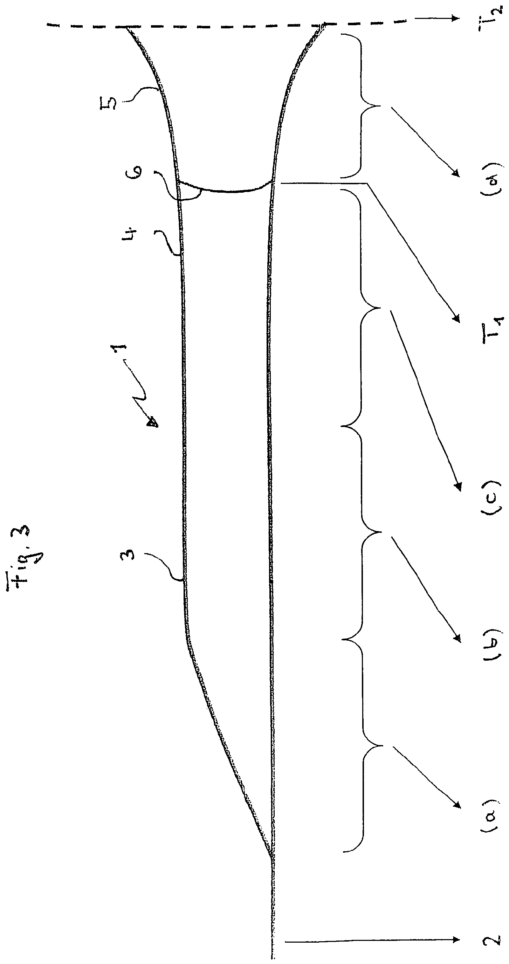

Figur 3 zeigt schematisch ein erfindungsgemäßes Implantat und seine Aufteilung in einzelne Abschnitte.

Das Implantat 1 hat einen proximalen Abschnitt (a), in dem sich das Implantat verjüngt und in einem Kupplungselement endet, hier als Draht dargestellt. Dieser Abschnitt entspricht dem Bereich 2 in Figur 2.

Daran schließt sich distal der Abschnitt (b) an, der der Fixierung des Implantats an der Gefäßwand des zuführenden Gefäßes Z dient. Dieser Bereich hat Maschen 3 mit einer relativ engen Maschenweite, die einen guten Kontakt zur Gefäßwandung herstellen.

Distal schließt sich dann der Abschnitt (c) an, der Maschen 4 mit einer relativ großen Weite aufweist. Dieser Bereich ist dazu bestimmt, einströmendes Blut in die Abzweigung X und Y, siehe Figur 1 und 2, abzugeben.

Das distale Ende des Implantats 1 ist der Abschnitt (d), in dem sich die Struktur 5 im gezeigten Fall trompetenförmig erweitert. Dieser Bereich kommt in dem Aneurysma A zu Liegen. Der Abschnitt (d) kann integraler Bestandteil des Implantats sein, d. h. zusammen mit den Abschnitten (a) bis (c) aus einem Rohr (Nitinol) geschnitten oder aus solchen Drähten geflochten sein. Es ist aber auch möglich, die Abschnitte (a) bis (c) aus einem Rohr zu schneiden und den Abschnitt (d) zu flechten und mit dem Abschnitt (c) zu verschweißen.

Zwischen den Abschnitten (c) und (d) ist die Trennzone T1 dargestellt, die ein oder mehrere Trennelemente 6 aufweist. Bei diesen Trennelementen kann es sich um eingespannte Fäden, Drähte oder Fasern, etwa aus Polyamid, handeln, aber auch um Teile einer geschnittenen Struktur, die nach innen umgeformt wurden. Diese Trennzone T1 mit den Trennelementen 6 dient dazu, in ein Aneurysma hinein praktizierte Okklusionsmittel zurückzuhalten.

Je nach Art des Aneurysmas kann die Trennzone auch in den Abschnitt (d) hineinverschoben sein oder sich sogar am distalen Ende des Abschnitts (d) befinden. Eine solche Trennzone T2 ist insbesondere dann sinnvoll, wenn die Bifurkation so umgewandelt ist, dass die abzweigenden Gefäße X und Y nicht direkt vom zuführenden Gefäß Z abzweigen, sondern aus dem Aneurysma abzweigen. In diesem Fall muss die Trennzone unmittelbar oberhalb der Abzweigungen im sich erweiternden Abschnitt des Implantats angeordnet sein. Der Abschnitt (d) ist auf das distale Ende des Implantats 1 beschränkt und geht in die Trennzone T2 über.

Figur 4 zeigt ein erfindungsgemäßes Implantat 1, wie es gemäß Figur 2 eingesetzt werden kann. Das Implantat 1 ist mit einem Führungsdraht 9 dargestellt und weist an seinem proximalen Ende 2 eine röntgendichte Markerspirale 7 auf. Das oder die Kupplungselemente, über die der Führungsdraht 9 mit dem Implantat 1 verbunden ist, sind nicht dargestellt, befinden sich aber im Bereich der Markerspirale 7.

Das dargestellte Implantat ist ein Geflecht aus einzelnen Drähten, die vorzugsweise aus Nitinol bestehen und denen die endgültige Implantatform aufgeprägt ist. Nitinol als Formgedächtnismaterial erlaubt es, das Implantat in komprimierter Form in einen Katheter zu führen ohne dass die Formgebung verlorengeht. Nach der Freisetzung aus dem Katheter nimmt das Implantat die ihm aufgeprägte Form an, so dass es seinen Einsatzzweck erfüllen kann.

Das Implantat 1 ist in vier Abschnitte (a) bis (d) gegliedert, wobei der Abschnitt (a) den sich verjüngenden proximalen Abschnitt darstellt, der im proximalen Ende 2 zusammenläuft und in dem oder den Kupplungselementen endet. Der Abschnitt (b) ist ein Fixierabschnitt, der an der Gefäßwandung des zuführenden Gefäßes Z zu Liegen kommt und relativ enge Maschen 3 aufweist. Der Abschnitt (c) ist durchlässig gestaltet mit weiteren Maschen 4, durch die der Blutstrom in die abzweigenden Gefäße X und Y austreten kann. Der Abschnitt (d) ist gegenüber dem Abschnitt (b) und hier auch gegenüber (c) erweitert und kommt im Aneurysma A zu Liegen. Die Enden der einzelnen Drähte sind durch Markerspiralen 8 aus einem röntgendichten Material, etwa Platin oder einer Platinlegierung, atraumatisch umgestaltet. Zwischen den Abschnitten (c) und (d) befindet sich ein Fasergeflecht 6, das beispielsweise aus Nylon gefertigt sein kann und das gleichzeitig die Trennzone T1 darstellt. Die Bezugsziffer 5 bezeichnet die sich nach außen erweiternden Maschen bzw. Filamente des Implantats 1 im distalen Bereich.

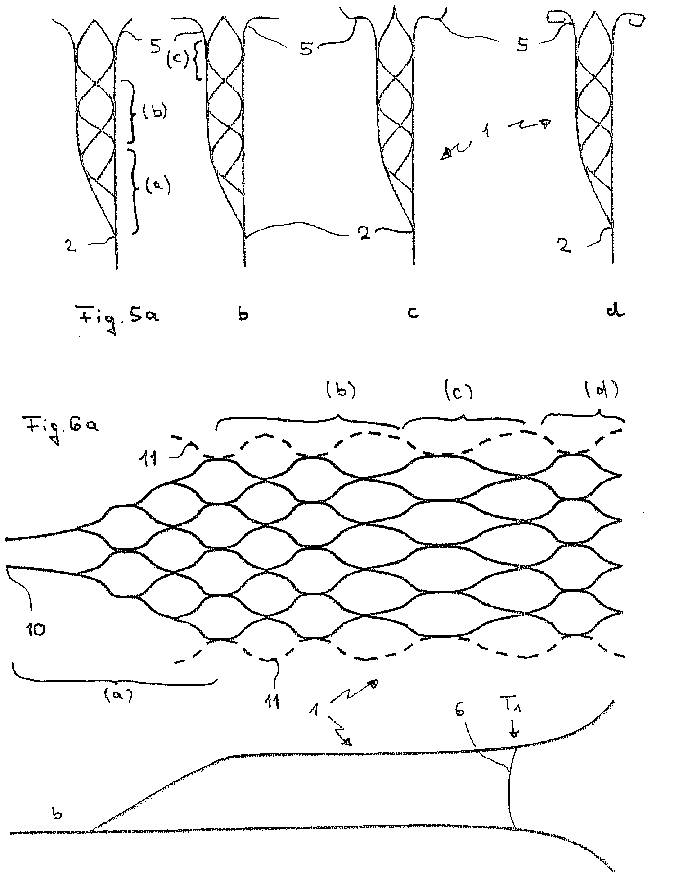

Figur 5 zeigt als Prinzipskizze vier Varianten der Ausgestaltung des distalen Bereiches 5 erfindungsgemäßer Implantate 1. Figur 5a zeigt ein sich trompetenförmig aufweitendes distales Ende des Implantats, d. h. der Abschnitt (d) weitet sich kelchförmig auf. Gemäß Figur 5b ist das distale Ende 5 tellerförmig aufgeweitet, mit einem sehr eng begrenzten distalen Abschnitt (d). Figur 5c zeigt eine Kombination der Designelemente von Figur 5a und 5b.

Figur 5d zeigt schließlich einen distalen Bereich mit eingerollten distalen Enden der einzelnen Filamente eines Implantats 1. Zur Orientierung sind in Figur 5a bzw. 5b die Abschnitte (a), (b), (c) eingezeichnet.

Figur 6 zeigt in flächig ausgebreiteter Form eine bevorzugte Ausführungsform eines erfindungsgemäßen Implantats 1 mit den Abschnitten (a) bis (d). Das Implantat 1 ist als aus Nitinolrohr geschnittene Maschenstruktur zu verstehen, wobei in der Darstellung die gestrichelt gezeichneten Stege 11 den ausgezogenen Stegen auf der gegenüberliegenden Seite entsprechen. Deutlich zu erkennen sind die vergrößerten Waben im Bereich des Abschnitts (c) in der Darstellung von Figur 6a sowie die kelch- oder trompetenförmige Erweiterung in der Prinzipskizze gemäß Figur 6b. Dort ist auch die Trennzone T1 mit Trennelementen in Form einer eingezogenen Ebene von Nylonfäden 6 dargestellt.

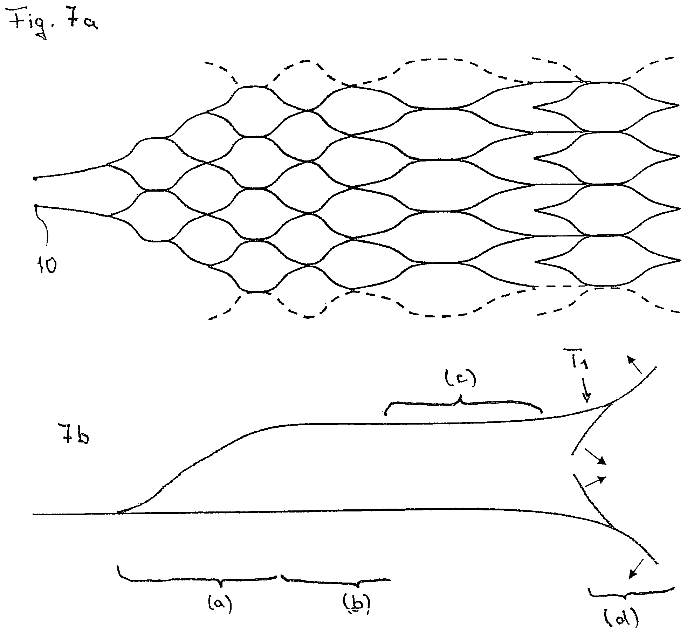

Figur 7 zeigt eine weitere Ausführungsform eines aus Nitinolrohr geschnittenen erfindungsgemäßen Implantats 1, mit den proximal angeordneten Kupplungselementen 10, einem sich nach proximal verjüngenden Abschnitt (a), einem Fixierabschnitt (b), dem Abschnitt mit größerer Maschenweite (c) und dem sich erweiternden Abschnitt (d). Das Implantat ist auch hier flächig dargestellt, entsprechend Figur 6, und weist die Besonderheit auf, dass Trennzone T1 aus aus dem Nitinolrohr ausgeschnittenen Elementen besteht, die bei der Umformung nach innen einklappen, während sich das distale Ende nach außen trompetenförmig erweitert. Die nach innen eingeklappten Stege 6 der Trennzone T1 dienen, wie in der Variante von Figur 6 die eingezogenen Nylonfäden, der Rückhaltung von in ein Aneurysma eingebrachten Okklusionsmitteln.

Die Implantate gemäß Figur 6 und 7 müssen keine rohrförmige Struktur haben, sondern können auch als gerollte "Matten" vorliegen, die sich gegen die Gefäßwand aufspannen. Auch eine partielle Schlitzung ist möglich.

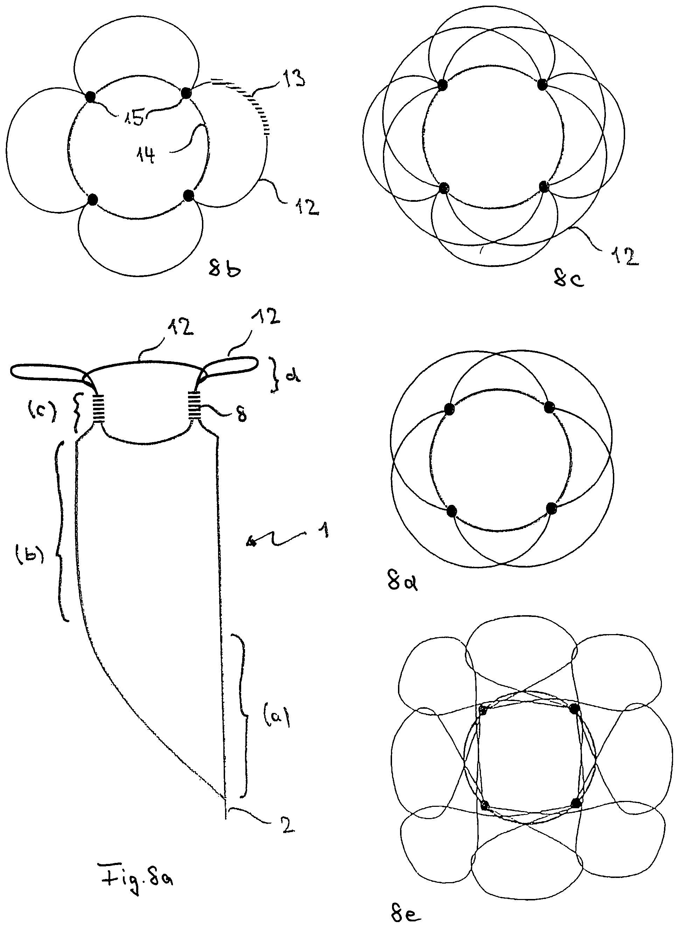

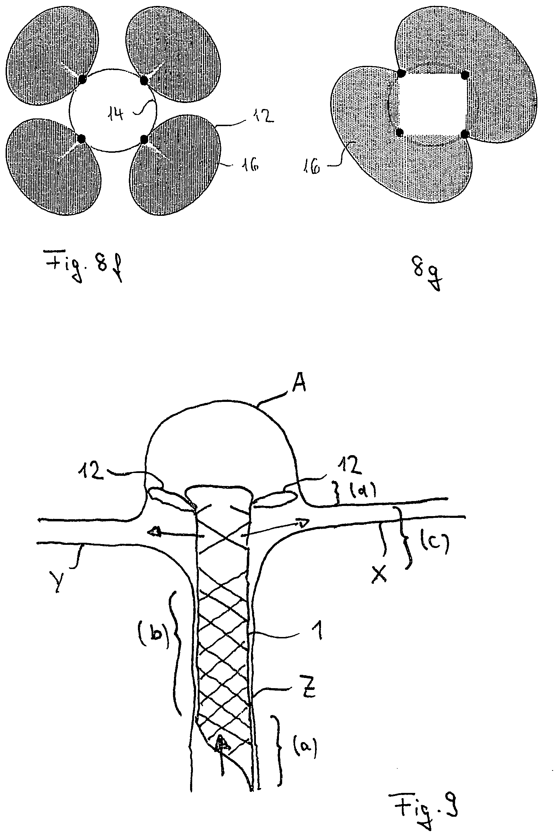

Figur 8 zeigt ein erfindungsgemäßes Implantat 1 mit einer eher tellerförmigen Ausgestaltung des Abschnitts (d), der im Wesentlichen aus Drahtschlaufen 12 besteht. Die Drahtschlaufen schließen an den zylindrischen Teil des Körpers des Implantats 1 an, wobei dieser zylindrische Teil von den Abschnitten (a) bis (c) gebildet wird. Im Übergangsbereich zu den angesetzten Schlaufen 12 befinden sich Markerelemente 8, die der sicheren Platzierung dienen. Im Bereich dieser Verbindung des zylindrischen Körpers des Implantats 1 und des Abschnitts (d) mit den Schlaufen 12 befindet sich der Abschnitt (c), der den Austritt des zufließenden Blutes in seitlich abgehende Gefäße erlaubt. Das Blut tritt somit zwischen den Stegen mit den Markerelementen 8 in die abgehenden Gefäße (X undY, Fig.2) ein.

Einzelne Varianten des distalen Abschnitts (d) sind in der Draufsicht in Figur 8b bis 8g dargestellt, wobei einzelne oder mehrere Schlaufen 12 mit Markerspiralen 13 versehen sein können. Die Markerspiralen 13 können die Schlaufen ganz oder teilweise umgeben. Die Schlaufen gehen im dargestellten Fall von vier Verbindungsstegen 15 aus, die auch die Markerelemente 8 tragen, wobei in den Darstellungen 9b bis 9g der innere Kreis 14 den Übergang in den zylindrischen Teil des Implantats darstellt. Etwaige vorhandene Verspannungen einer Trennzone T1 oder T2 sind nicht dargestellt.

Die Ausführungsformen gemäß Figur 8f und g zeigt mit einer dehnfähigen Membran 16 versehene Schlaufen 12, die in diesem Fall gleichzeitig eine Trennzone T2 ausbilden, wie in Figur 3 gezeigt.

Es versteht sich, dass die Trennzonen T1 und T2 den zu okkludierenden Abschnitt des Aneurysmas A abteilen müssen. Je nach Typ des Aneurysmas liegt diese Trennzone dann im Eingangsbereich - bei proximal vom Eingangsbereich abgehenden Zweiggefäßen - oder aber innerhalb des Aneurysmas - dann, wenn zwei Gefäße aus dem Aneurysmaraum selbst abzweigen - wobei im letzteren Fall nur der von abzweigenden Gefäßen freie Teil des Aneurysmas okkludiert werden kann. Insbesondere bei tellerförmig ausgebildeten distalen Abschnitten (d) der erfindungsgemäßen Implantate kann sich, insbesondere bei größerer Anzahl der Drahtschlaufen, eine zusätzliche Verspannung oder Anordnung aus dem Rohr geschnittenen Trennelementen erübrigen.

Die in Figur 8 dargestellten schlaufenförmigen distalen Abschnitte (d) können zum einen, wie auch der übrige Implantatkörper, aus einem Rohr mit dafür geeignetem Durchmesser geschnitten werden. Es ist aber auch möglich, die Abschnitte (a) bis (c) des Implantatkörpers aus einem Rohr in üblicher Art und Weise zu schneiden und den Abschnitt (d) aus Drahtfilamenten anzuheften, beispielsweise durch Laserverschweißen.

Figur 9 zeigt den Spezialfall eines Aneurysmas A, bei dem die abzweigenden Gefäße X und Y aus dem Aneurysma abgehen. Für diesen Fall sind die in Figur 8 beschriebenen Implantate 1 besonders geeignet, bei denen die Schlaufen 12 gleichzeitig die Trennzone T2 bilden, die im Aneurysma selbst distal von den abzweigenden Gefäßen angeordnet sind. Der zylindrische Körper des Implantats 1 mit den Abschnitten (a) und (b) befindet sich im zuführenden Gefäß Z, der Abschnitt (c), der den Durchtritt des Bluts in die Abzweigungen X und Y erlaubt, liegt im Bereich dieser Abzweigung und mittelbar distal dieses Abschnitts (c) befindet der Abschnitt (d) mit den Schlaufen 12. Die Schlaufen können mit einer Membran bespannt sein, wobei diese Membran aus einem dehnfähigen Material besteht, beispielsweise Teflon, oder einem Faservlies. Ein solches Faservlies aus Polycarbonaturethan ist aus der DE 28 06 030 bekannt und zeichnet sich durch eine hohe Elastizität aus, die für die Applikation des Implantats durch einen Katheter günstig ist. Die Membran kann geschlitzt, gefaltet oder porös gestaltet sein, beispielsweise auch um Material einzusparen und den Transport durch einen Katheter zu erleichtern.

Eine derartige Membran kann auch als Trennelement für die Trennzone verwandt werden, wie sie zwischen den Abschnitten (c) und (d) angeordnet ist.

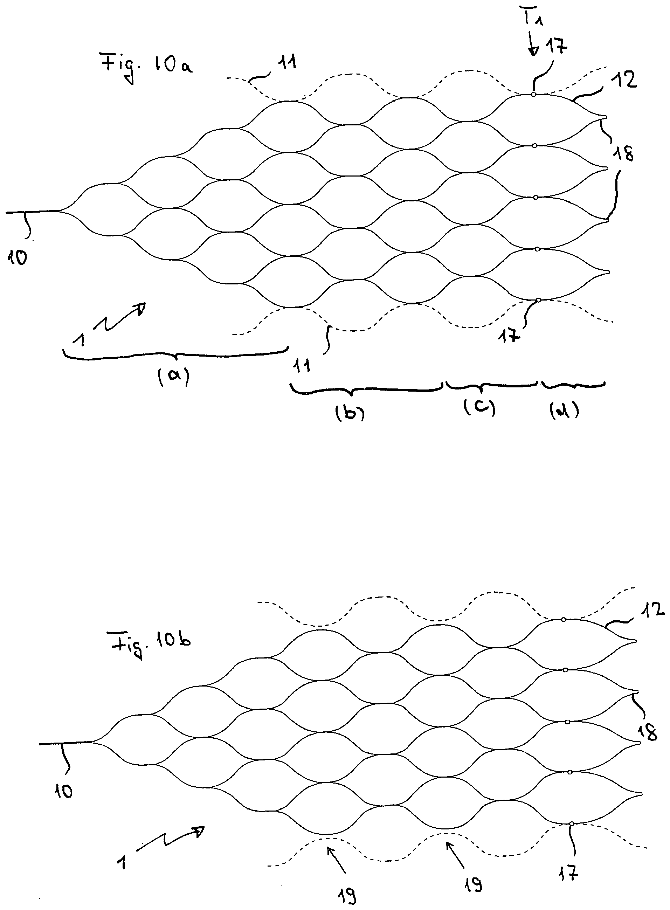

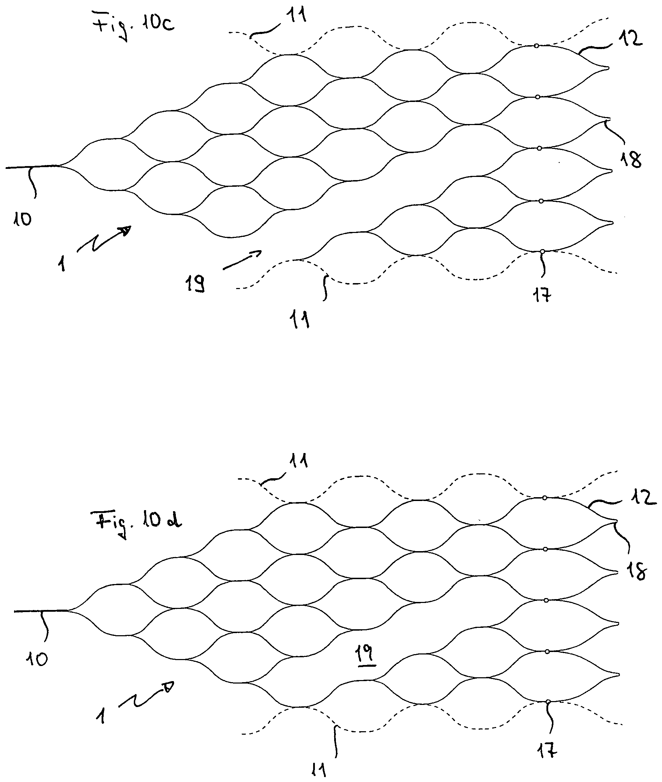

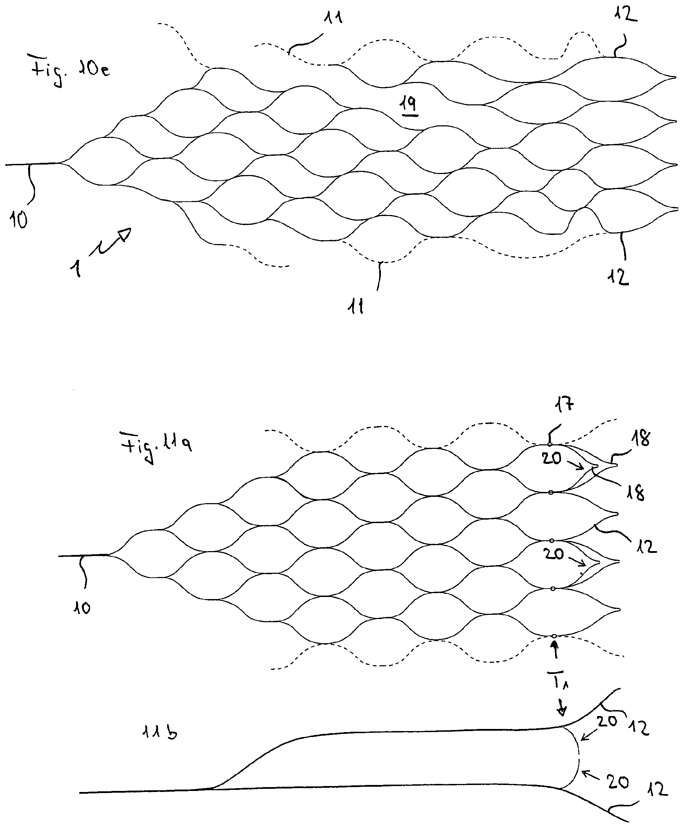

Figur 10 zeigt in flächig ausgebreiteter Form mehrere bevorzugte Ausführungsformen eines erfindungsgemäßen Implantats 1, bei der die Wabenstruktur durch im wesentlichen gleichmäßig große warm ausgebildet wird; lediglich die distalen Schlaufen haben eine größere Wabenfläche.

Wie in Figur 6 entsprechen die gestrichelt gezeichneten Stege 11 den ausgezogenen Stegen auf der gegenüberliegenden Seite. Das Implantat 1 entspricht damit einem Rohr mit einer Gitter- oder Wabenstruktur.

An das proximal angeordnete Kupplungselement 10 schließt sich der proximale Abschnitt (a) daran der Fixierabschnitt (b) an. Der distale Abschnitt (d) beginnt im Bereich der Ösen 17, die zur Aufnahme und Fixierung von Draht- oder Nylonelementen dienen, mit denen in das Implantat eine Trennebene eingezogen wird. Die distalen Schlaufen im sich nach außen erweiternden Abschnitt (d) weisen distal Nasen auf, die sich als vorteilhaft bei der Einführung des Implantats durch einen Katheter an dem Einsatzort erwiesen haben.

Figur 10b entspricht in allen wesentlichen Punkten der Darstellung von Figur 10a, ausgenommen einer partiellen Schlitzung im Bereich der Pfeile 19, wo die Rohrstruktur des Implantats 1 nicht geschlossen ist. Die Schlitzung verläuft achsparallel und endet vor dem distalen Abschnitt (d), dort wo sich der durchlässige Abschnitt (c) befindet.

Figur 10c zeigt eine Variante mit einem nicht achsparallel verlaufenden Schlitz 19, der sich um die Längsachse windet, ebenfalls jedoch vor dem distalen Abschnitt (d) endet.

Derartige Schlitzungen haben sich als ausgesprochen vorteilhaft für die Flexibilität im Bereich der Fixierzone (b) erwiesen. Die Radialkraft des Implantats 1 welche hierdurch nicht wesentlich beeinträchtigt ist, jedoch die Anpassung an den Gefäßverlauf und das Gefäßlumen verbessert.

Figur 10d zeigt ebenfalls ein erfindungsgemäßes Implantat mit Schlitzung, wobei jedoch die Schlitzung sich nicht bis zu den Implantaträndern erstreckt.

Figur 10e zeigt eine weitere Variante mit einem Schlitz 19, der sich ebenfalls um die Längsachse windet, wobei allerdings Wabenformen nebeneinander existieren. Die Wabenform hat einen Einfluss auf die Flexibilität und kann je nach Anforderung gewählt werden.

Die Schlaufen bzw. Waben des distalen Abschnitts (d) sind in Figur 10 jeweils mit der Bezugsziffer 12 bezeichnet.

Figur 11 zeigt eine weitere Variante eines erfindungsgemäßen Implantats 1 mit einem einzelnen Kupplungselement 10 und einer im wesentlichen regelmäßigen Wabenstruktur, bei dem als Trennelemente zusätzliche Schlaufen 20 vorgesehen sind. In dieser Hinsicht entspricht die Ausführungsform von Figur 11 der Ausführungsform von Figur 7. Die zusätzlichen Schlaufen 20 sind beim implantierten Produkt nach innen gerichtet und bilden die Trennebene T1 aus. Auch diese Schlaufen 20 weisen Nasen 18 auf, die den Transport durch einen Katheter erleichtern.

Figur 11b zeigt schematisch das Implantat von Figur 11a mit den einwärts weisenden Schlaufen 20 und der Trennebene T1.

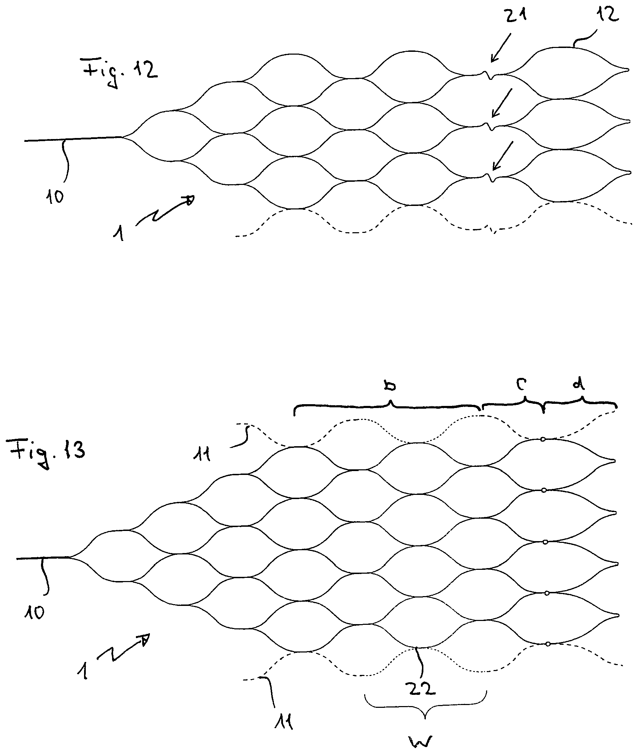

Figur 12 zeigt eine weitere Variante eines besonders flexiblen Implantats 1 mit Gelenkverbindern 21 in Form einer Zickzackführung der entsprechenden Stege zur Verbesserung der Anpassung des Implantats 1 an gekrümmt verlaufende Gefäße im Bereich der Bifurkation.

Figur 13 zeigt schließlich eine weitere Variante, bei der im Abschnitt (b) ein Teil der Waben mit dünneren Stegen ausgebildet ist (Bereich W), um die Flexibilität und Biegefähigkeit zu erhöhen. Dieser Bereich 22 liegt in der Fixierzone (b) und soll einem nicht regelmäßigen Verlauf eines Gefäßes in der Fixierzone Rechnung tragen. Im Übrigen entspricht das Implantat 1 den zuvor dargestellten Varianten.

## Patentansprüche

1. Implantat (1) zum Einsatz bei der Okkludierung von Aneurysmen im Bereich von Gefäßverzweigungen, insbesondere Bifurkationsaneurysmen (A) mit einer Maschenstruktur (3, 4), welches - von proximal nach distal - die Abschnitte (a) bis (d) aufweist:
(a) einen sich verjüngenden proximalen Abschnitt, in dem die Maschenstruktur zu einem oder mehreren Kupplungselementen (10) zusammengeführt ist,
(b) einen Fixierabschnitt, mit dem das Implantat an einer Gefäßwand abstützbar ist,
(c) einen durchlässigen Abschnitt mit einer größeren Maschenweite als in Abschnitt (b) für den Bereich der Gefäßbifurkation und
(d) einen distalen Abschnitt, in dem das Implantat gegenüber den Abschnitt (b) erweitert ist und der zur Platzierung im Aneurysma (A) bestimmt ist,
wobei, zwischen den Abschnitten (c) und (d) eine Trennzone (T1) angeordnet ist und die Trennzone (T1) Trennelemente aus quer zum Implantat (1) im Wesentlichen in einer Ebene verlaufenden Filamenten (6) aufweist, **dadurch gekennzeichnet, dass** der distale Abschnitt (d) aus einer Mehrzahl von mit dem Abschnitt (c) verbundenen Schlaufen (12) besteht, die gerundete und atraumatische Nasen (18) am distalen Ende aufweisen, wobei der distale Abschnitt (d) trompetenförmig oder korbförmig durch die Schlaufen (12) erweitert ist.

2. Implantat nach Anspruch 1 , **dadurch gekennzeichnet, dass** es aus Formgedächtnismaterial besteht.

3. Implantat nach einem der vorstehenden Ansprüche, **dadurch gekennzeichnet, dass** es zumindest partiell aus einem Rohr geschnitten ist.

4. Implantat nach einem der vorstehenden Ansprüche, **dadurch gekennzeichnet, dass** der proximale Abschnitt (a) in zwei Kupplungselementen (10) ausläuft.

5. Implantat nach einem der vorstehenden Ansprüche, **dadurch gekennzeichnet, dass** der distale Abschnitt (d) atraumatisch, weich und elastisch ausgebildet ist.

6. Implantat nach Anspruch 1, **dadurch gekennzeichnet, dass** die Enden der Drähte (12) im distalen Abschnitt (d) atraumatisch zusammengeführt sind.

7. Implantat nach einem der vorstehenden Ansprüche, **dadurch gekennzeichnet, dass** es ein seitlich geschlossenes rohrförmiges Design hat.

8. Implantat nach einem der Ansprüche 1 bis 6, **dadurch gekennzeichnet, dass** es partiell oder durchgehend geschlitzt ist..

9. Implantat nach einem der vorstehenden Ansprüche, **dadurch gekennzeichnet, dass** in dem distalen Abschnitt (d) Markerelemente (8) angeordnet sind.

10. Implantat nach einem der vorstehenden Ansprüche, angekoppelt an einen Führungsdraht (9).

## Claims

1. Implant (1) to be used for the occlusion of aneurysms in the region of vessel branches, in particular bifurcation aneurysms (A), with a mesh structure (3, 4), said implant comprising - from proximal to distal - sections (a) to (d):
(a) is a section tapering down proximally in which the mesh structure is brought together to form one or several coupling elements (10),
(b) is a fixing section by means of which the implant can be supported on the wall of a vessel,
(c) is a permeable section with a mesh width bigger than in section (b) for the region of the vessel bifurcation, and
(d) is a distal section in which the implant is expanded in comparison to section (b) and which is intended for placement into the aneurysm (A),
wherein a separation zone (T1, T2) is arranged between the sections (c) and (d), and the separation zone (T1, T2) is provided with separation elements consisting of filaments (6) extending substantially in a plane crosswise to the implant (1), **characterized in that**
the distal section (d) consists of a plurality of loops (12) which are connected with section (c) and which are provided at the distal end with rounded and atraumatic spouts (18), wherein the distal section (d) is widened out in a trumpet- or basket-like shape by means of loops (12).

2. Implant according to claim 1, **characterized in that** it consists of shape-memory material.

3. Implant according to any one of the above claims, **characterized in that** the implant at least partially is cut out of a tube.

4. Implant according to any one of the above claims, **characterized in that** the proximal section (a) terminates in two coupling elements (10).

5. Implant according to any one of the above claims, **characterized in that** the distal section (d) is designed so as to be atraumatic, soft, and elastic.

6. Implant according to claim 1, **characterized in that** the loops (12) in the distal section (d) are merged atraumatically.

7. Implant according to any one of the above claims, **characterized in that** it is of laterally closed tubular design.

8. Implant according to any one of claims 1 to 6, **characterized in that** the implant is partially or continuously slotted.

9. Implant according to any one of the above claims, **characterized in that** marker elements (8) are arranged in the distal section (d).

10. Implant according to any one of the above claims which is coupled to a guidewire (9).

## Revendications

1. Implant (1) à utiliser pour l'occlusion d'anévrismes dans la région de ramifications de vaisseaux, en particulier d'anévrismes de bifurcation (A) avec une structure maillée (3, 4), qui présente - d'une extrémité proximale à une extrémité distale - les parties (a) à (d) :
(a) une partie proximale qui se rétrécit, dans laquelle la structure maillée se rassemble en un ou plusieurs élément(s) de couplage (10),
(b) une partie de fixation, avec laquelle l'implant peut être appuyé sur une paroi de vaisseau,
(c) une partie perméable avec une plus grande largeur de mailles que dans la partie (b) pour la région de la bifurcation du vaisseau, et
(d) une partie distale, dans laquelle l'implant est élargi par rapport à la partie (b) et qui est destinée à être placée dans l'anévrisme (A),
dans lequel une zone de séparation (T1) est disposée entre les parties (c) et (d) et la zone de séparation (T1) présente des éléments de séparation constitués de filaments (6) s'étendant essentiellement dans un plan transversalement à l'implant (1),
**caractérisé en ce que** la partie distale (d) se compose d'une multiplicité de boucles (12) reliées à la partie (c), qui présentent à l'extrémité distale des nez arrondis et atraumatiques (18), dans lequel la partie distale (d) est élargie en forme de trompette ou en forme de panier par les boucles (12).

2. Implant selon la revendication 1, **caractérisé en ce qu'**il est constitué d'un matériau à mémoire de forme.

3. Implant selon l'une quelconque des revendications précédentes, **caractérisé en ce qu'**il est au moins en partie découpé hors d'un tube.

4. Implant selon l'une quelconque des revendications précédentes, **caractérisé en ce que** la partie proximale (a) se termine en deux éléments de couplage (10).

5. Implant selon l'une quelconque des revendications précédentes, **caractérisé en ce que** la partie distale (d) est réalisée sous forme atraumatique, souple et élastique.

6. Implant selon la revendication 1, **caractérisé en ce que** les extrémités des fils (12) sont rassemblées de façon atraumatique dans la partie distale (d).

7. Implant selon l'une quelconque des revendications précédentes, **caractérisé en ce qu'**il présente un design tubulaire latéralement fermé.

8. Implant selon l'une quelconque des revendications 1 à 6, **caractérisé en ce qu'**il est fendu de façon partielle ou continue.

9. Implant selon l'une quelconque des revendications précédentes, **caractérisé en ce que** des éléments de marqueur (8) sont disposés dans la partie distale (d).

10. Implant selon l'une quelconque des revendications précédentes, accouplé à un fil de guidage (9).
